# EUROPÄISCHE PATENTSCHRIFT

(11) **EP 3 146 453 B1**
(45) Veröffentlichungstag und Bekanntmachung des Hinweises auf die Patenterteilung: **29.07.2020**
(21) Anmeldenummer: 15735878.9
(22) Anmeldetag: 26.06.2015
(51) Int. Cl.: G16H 10/60

(54) **MEDIZINGERÄTESTEUERUNGSSYSTEM UND VERFAHREN ZUM BETREIBEN DES MEDIZINGERÄTESTEUERUNGSSYSTEMS**
MEDICAL DEVICE CONTROL SYSTEM, AND METHOD FOR OPERATING SAID MEDICAL DEVICE CONTROL SYSTEM
SYSTÈME DE COMMANDE D'APPAREILLAGE MÉDICAL ET PROCÉDÉ POUR LE FAIRE FONCTIONNER

(30) Priorität: 30.06.2014 DE 102014212658
(43) Veröffentlichungstag der Anmeldung: 29.03.2017
(73) Patentinhaber: TRUMPF Medizin Systeme GmbH + Co. KG, 07318 Saalfeld (DE)
(72) Erfinder: MARKA, Rudolf, 85737 Ismaning (DE); ÖZHAN, Serhan, 81379 München (DE); HAN, Gel, 80802 München (DE)
(74) Vertreter: Prüfer & Partner mbB Patentanwälte · Rechtsanwälte
(86) Internationale Anmeldenummer: PCT/EP2015/064538
(87) Internationale Veröffentlichungsnummer: WO 2016/001086

(56) Entgegenhaltungen:
- WO-A1-2008/016580
- US-A1- 2002 109 578
- US-A1- 2005 091 338
- US-A1- 2005 105 734
- "Tegris reDeFiNiNg Or iNTegrATiON", , 1. Oktober 2012 (2012-10-01), XP55129429, Rastatt, Germany Gefunden im Internet: URL:http://www.maquet.com/content/Surgical Workplaces/Documents/Brochures/Internet_TE GRIS_Brochure_10000911-EN-1B_NONUS.pdf [gefunden am 2014-07-16]

## Beschreibung

Die Erfindung betrifft ein Medizingerätesteuerungssystem und ein Verfahren zum Betreiben des Medizingerätesteuerungssystems, insbesondere ein System und ein Verfahren, das eine vereinfachte Freigabe einer Bedienfunktion ermöglicht.

Medizingeräte wurden bislang mittels Bedienfelder gesteuert, die an den Medizingeräten angebracht waren, oder mittels einer dem Medizingeräte zugeordneten Fernbedienung. Dabei war eine Autorisierung für eine Bedienung der einzelnen Medizingeräte klar abgegrenzt.

In letzter Zeit werden verstärkt mobile Steuerungseinheiten für die Bedienung von mehreren Medizingeräten verwendet. Dabei ist es aber gegebenenfalls erforderlich, dass nur spezielle Personen zu der Bedienung der Medizingeräte autorisiert werden.

Eine aufwändige Anmeldeprozedur ist aber beispielsweise in einem Operationssaal während einer Operation nicht möglich, da in einer Notsituation eine sofortige Bedienung des Medizingeräts erforderlich sein kann.

Dokument WO 2008016580 A1 offenbart eine Einrichtung und ein Verfahren zum Verwalten eines Nutzerzugriffs zu einer Computerumgebung. Bei einer ersten Anmeldung eines Nutzers wird eine Berechtigungsnachweisinformation eingegeben, um eine Identität des Nutzers zu authentifizieren, und eine physische Einheit mit Identifikationsinformationen wird einer Benutzerschnittstelle vorgehalten, um die physische Einheit mit dem durch die eingegebene Berechtigungsnachweisinformation authentifizierten Nutzer zu verbinden. Wenn der Nutzer eine nachfolgende Anmeldung tätigt, hält er lediglich die physische Einheit vor die Benutzerschnittstelle, um den Zugriff zu erlangen.

In Dokument US 2005/0105734 A1 ist ein Authentifikationssystem gezeigt, das Zugriff zu gesicherten Diensten einer Rechnereinrichtung ermöglicht. Der Nutzer erhält nur Zugriff zu dem Dienst, wenn ein dem Nutzer zugeordnetes drahtloses Token in der Nähe der Rechnereinrichtung ist.

Auch Dokument US 2002/0109578 A1 offenbart ein Identifikationssystem, in dem eine Identifikation eines Nutzers über einen Transponder mit einem darin programmierten Identifikationscode erfolgt.

Der Erfindung liegt die Aufgabe zu Grunde, ein Verfahren für ein Medizingerätesteuerungssystem bereitzustellen, das eine vereinfachte Anmeldeprozedur zum Betreiben des Medizingerätesteuerungssystems für vorbestimmte Bediener ermöglicht.

Die Aufgabe wird mit einem Verfahren gemäß Anspruch 1 gelöst.

Durch ein Medizingerätesteuerungssystem gemäß Anspruch 1 ist es möglich eine einfache Freigabe einer Bedienung eines mobilen Steuerungssystems personenbezogen zu ermöglichen.

Die Erfindung wird nun anhand von Ausführungsbeispielen unter Bezugnahme auf die beigefügten Zeichnungen erläutert.

Insbesondere zeigen:
- Fig. 1: eine perspektivische Ansicht eines Operationssaals; und
- Fig. 2: einen Grundriss eines Gebäudebereichs mit Operationssälen.

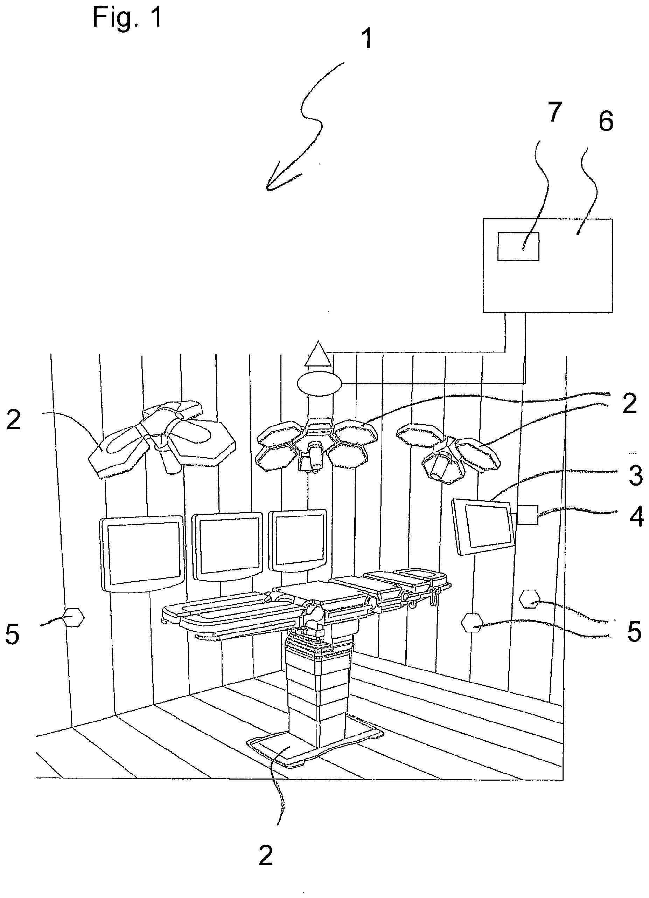

In Fig. 1 ist eine perspektivische Ansicht eines Operationssaals als Raum 1 in einem Gebäudebereich, beispielsweise in einem Krankenhaus, gezeigt. In dem Raum 1 sind ein Operationstisch und mehrere Operationsleuchten als Medizingeräte 2 dargestellt. Ferner ist ein Tablet-PC als eine mobile Steuerungseinheit 3 für die Medizingeräte 2 vorgesehen. Die mobile Steuerungseinheit 3 ist mit einem Funk-Empfangsmodul 4 ausgestattet.

Ferner sind in dem Raum 1 separate mobile Funk-Sendemodule 5 vorhanden. Die separaten mobilen Funk-Sendemodule 5 sind in sogenannten badges vorgesehen. Diese badges werden hier vom Operationspersonal getragen. Die Funk-Sendemodule 5 senden jeweils ein Signal aus, das ein den einzelnen badges entsprechendes Identifikationsmerkmal aufweist, mit dem die einzelnen Personen des Operationspersonals identifiziert werden können. Wie in Fig. 1 zu sehen ist, befindet sich ein badge 5 am linken Rand der Figur und zwei badges 5 unmittelbar an der mobilen Steuerungseinheit 3.

Die von den separaten mobilen Funk-Sendemodulen 5 ausgesendeten Signale werden von dem Funk-Empfangsmodul 4 der mobilen Steuerungseinheit 3 empfangen, so dass die Funk-Sendemodule 5 in der Lage sind, mit dem Funk-Empfangsmodul 4 zu kommunizieren.

Weiterhin ist eine Steuerungsvorrichtung 6 vorgesehen, die mit der mobilen Steuerungseinheit 3 verbunden ist. Die Steuerungsvorrichtung 6 weist einen Speicherbereich 7 auf, in dem die Identifikationsmerkmalen der badges bzw. der Funk-Sendemodule 5 gegebenenfalls in Verbindung mit weiteren Daten, hier optional mit einem Kennwort oder einem später beschriebenen Lang-Kennwort, gespeichert sind.

Die Steuerungsvorrichtung 6 ist so ausgebildet, dass sie ein von dem Funk-Empfangsmodul 4 erfasstes und an die Steuerungsvorrichtung 6 weitergeleitetes Signal mit dem Identifikationsmerkmal erfasst und es mit den in dem Speicherbereich 7 gespeicherten Identifikationsmerkmalen vergleicht. Nach einem positiven Vergleichsergebnis, wenn eine Übereinstimmung der Identifikationsmerkmale festgestellt wurde, ist die Steuerungsvorrichtung 6 ausgebildet, eine Bedienfunktion der mobilen Steuerungseinheit 3 freizugeben.

Optional erfolgt diese Freigabe erst nach einer Eingabe eines zugehörigen Kennworts oder Lang-Kennworts.

Das Funk-Empfangsmodul 4 ist so angepasst, dass es eine Signalstärke des Funksignals des Funk-Sendemoduls 5 erfasst. In dem Funk-Empfangsmodul 4 ist ein Schwellwert festgelegt, so dass nur Funksignale, deren Signalstärke über dem Schwellwert liegen, von dem Funk-Empfangsmodul 4 an die Steuerungsvorrichtung 6 weitergeleitet werden.

Diese Komponenten, die mobile Steuerungseinheit 3, das Funk-Empfangsmodul 4, das Funk-Sendemodul 5 und die Steuerungsvorrichtung 6, sind Bestandteile eines Medizingerätesteuerungssystems.

Das Funk-Empfangsmodul 4 der mobilen Steuerungseinheit 3 und das Funk-Sendemodul 5 sind in diesem Ausführungsbeispiel "Bluetooth-Low-Energy" (BLE)-Module. In alternativen Ausführungsformen sind diese Module beispielsweise RFID-Module, ZigBee-Module oder WLAN-Module.

In diesem Ausführungsbeispiel sind eine einzelne mobile Steuerungseinheit 3 und vier Medizingeräte 2, nämlich der Operationstisch und drei Operationsleuchten, dargestellt. In alternativen Ausführungsformen können auch mehrere mobile Steuerungseinheiten 3 und/oder eine andere Anzahl von Medizingeräten 2 vorgesehen sein.

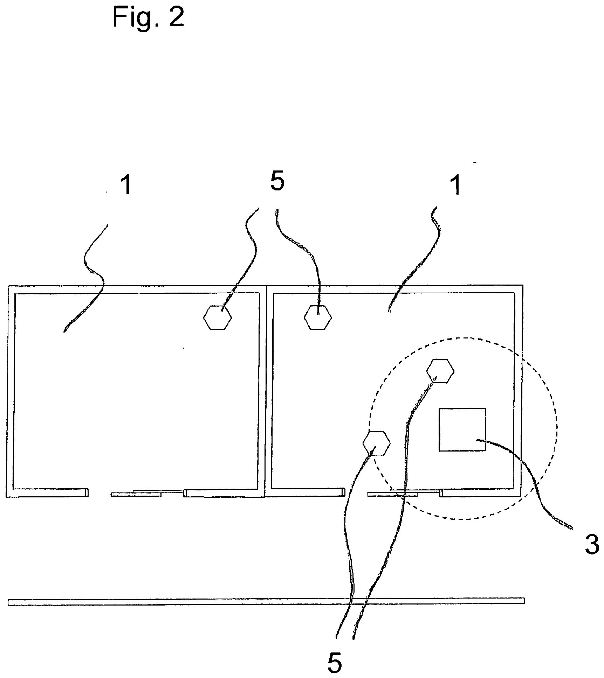

In Fig. 2 ist ein Grundriss des Gebäudebereichs mit den Operationssälen als den Räumen 1 gezeigt.

Wie in Fig. 1 zu sehen, befinden sich zwei badges mit den Funk-Sendemodulen 5 unmittelbar an der mobilen Steuerungseinheit 3 und ein badge mit einem der Funk-Sendemodule 5 in der gegenüberliegenden Ecke des Raums 1. Es befinden sich also zwei Mitglieder des Operationspersonals in unmittelbarer Reichweite der mobilen Steuerungseinheit 3. Ein weiteres badge mit einem der Funk-Sendemodule 5, also ein weiteres Mitglied des Operationspersonals, befindet sich in einem anderen Raum 1. Um die mobile Steuerungseinheit 3 herum ist ein Bereich mit einem maximalen vorbestimmten Abstand angegeben, in dem sich die Funk-Sendemodule 5 befinden müssen, so dass ihre Signalstärke über dem Schwellwert liegt. Somit wird ihre entsprechende Anzeige auf der mobilen Steuerungseinheit 3 angezeigt.

Um das Medizingerätesteuerungssystem zu bedienen, muss im Betrieb ein Bediener zuerst angemeldet werden. Um die Anmeldung möglichst rasch durchzuführen, werden die Identifikationsmerkmale gegebenenfalls zusammen mit einem verkürzten Kennwort verwendet.

Dazu sendet das Funk-Sendemodul 5 der badges in periodischen Abständen eine Nachricht mit dem Identifikationsmerkmal des Funk-Sendemoduls 5. Dieses Identifikationsmerkmal ist beispielsweise eine MAC-Adresse oder ein Name des Moduls. Das Signal wird von dem Funk-Empfangsmodul 4 in der mobilen Steuerungseinheit 3 empfangen und an die Steuerungsvorrichtung 6 weitergeleitet. Die Steuerungsvorrichtung 6 vergleicht das Identifikationsmerkmal mit den in dem Speicherbereich 7 gespeicherten Identifikationsmerkmalen und gibt bei einem positiven Vergleichsergebnis, wenn also das erfasste Identifikationsmerkmal mit einem der gespeicherten Identifikationsmerkmale übereinstimmt, die Bedienfunktion der mobilen Steuereinheit 3 frei.

Optional wird diese Freigabe nach einer vorbestimmten Zeitdauer, in der die mobile Steuerungseinheit 3 nicht bedient wird, wieder aufgehoben.

Sobald die Freigabe nach der vorbestimmten Zeitdauer wieder aufgehoben wird oder sich der Bediener abmeldet, werden die Signale der Funk-Sendemodule 5 wieder erfasst und die Umgebung der mobilen Steuerungseinheit 3 quasi abgesucht.

In einer weiteren Option ist es erforderlich, zusätzlich ein Kennwort einzugeben. Dazu wird auf der mobilen Steuerungseinheit 3 eine dem Identifikationsmerkmal entsprechende Anzeige, beispielsweise ein Name eines Mitglieds des Operationspersonals, ausgegeben und das entsprechende Kennwort dazu wird von der entsprechenden Person an der mobilen Steuerungseinheit 3 eingegeben.

In alternativen Ausführungsformen ist es auch möglich, anstatt des Kennworts, ein graphisches Muster einzugeben oder eine Gesichts- oder Fingerabdruckerkennung durchzuführen.

Wenn sich mehrere Mitglieder des Operationspersonals mit badges im Bereich der mobilen Steuerungseinheit 3 befinden, werden optional sämtliche den Identifikationsmerkmalen entsprechende Anzeigen (Namen) ausgegeben. Ein Mitglied des Operationspersonals wählt dann seinen Namen und gibt sein entsprechendes Kennwort ein.

Um eine größere Sicherheit zu gewährleisten ist es optional bei einer erstmaligen Anmeldung an dem Medizingerätesteuerungssystem erforderlich ein sogenanntes Lang-Kennwort einzugeben. Dieses Kennwort entspricht dann entsprechenden Vorgaben bezüglich seiner Sicherheit und weist eine entsprechende Länge auf. Sobald die erstmalige Anmeldung erfolgt ist, kann ein kürzeres Kennwort eingegeben werden, um in Kombination mit dem Identifikationsmerkmal einen sogenannten Quick-Login durchzuführen.

Die Mitglieder des Operationspersonals, die sich in der Nähe der mobilen Steuerungseinheit 3 befinden, werden als mögliche Bediener des Medizingerätesteuerungssystems betrachtet. Diese Betrachtungsweise wird in dem Medizingerätesteuerungssystem so umgesetzt, dass der Schwellwert in dem Funk-Empfangsmodul 4 so eingestellt wird, dass nur die entsprechenden Anzeigen von in einem vorbestimmten Abstand von der mobilen Steuerungseinheit 3 befindlichen Funk-Sendemodule 5 gezeigt werden. Als vorbestimmter Abstand kann beispielsweise der Aktionsbereich eines der Bediener angenommen werden.

In einer weiteren alternativen Ausführungsform werden nur bestimmte Bedienfunktionen der mobilen Steuerungseinheit 3, die einem Identifikationsmerkmal, also einem der Bediener zugeordnet sind, freigegeben.

Die verschiedenen Ausführungsformen mit den einzelnen Optionen sind untereinander kombinierbar.

## Patentansprüche

1. Verfahren zum Betreiben eines Medizingerätesteuerungssystems, aufweisend
mindestens eine mobile Steuerungseinheit (3) für mindestens ein Medizingerät (2), mit zumindest einem Funk-Empfangsmodul (4) zum Erfassen und Weiterleiten von Funksignalen,
eine mit der mobilen Steuerungseinheit (3) verbundene Steuerungsvorrichtung (6), und
zumindest ein separates mobiles Funk-Sendemodul (5), das in der Lage ist, mit dem Funk-Empfangsmodul (4) zu kommunizieren,
wobei das zumindest eine Funk-Sendemodul (5) ausgebildet ist, ein Identifikationsmerkmal an das Funk-Empfangsmodul (4) zu senden, und
die Steuerungsvorrichtung (6) ausgebildet ist, ein von dem Funk-Empfangsmodul (4) erfasstes und an die Steuerungsvorrichtung (6) weitergeleitetes Signal mit dem Identifikationsmerkmal zu erfassen, das Identifikationsmerkmal mit gespeicherten Identifikationsmerkmalen zu vergleichen und nach einem positiven Vergleichsergebnis eine Bedienfunktion der mobilen Steuerungseinheit (3) freizugeben, wobei
das mindestens eine Funk-Sendemodul (5) ein Signal mit seinem Identifikationsmerkmal sendet,
das Funk-Empfangsmodul (4) das Signal empfängt und an die Steuerungsvorrichtung (6) weiterleitet,
die Steuerungsvorrichtung (6) das Identifikationsmerkmal mit gespeicherten Identifikationsmerkmalen vergleicht, und
die Steuerungsvorrichtung (6) die Bedienfunktion der mobilen Steuerungseinheit (3) freigibt, wenn das Vergleichsergebnis positiv ist,
**dadurch gekennzeichnet, dass** zu dem Identifikationsmerkmal jedes Funk-Sendemoduls (5) ein entsprechendes Kennwort gespeichert ist, und die mobile Steuerungseinheit (3) eine dem Identifikationsmerkmal entsprechende Anzeige ausgibt und die Bedienfunktion der mobilen Steuerungseinheit (3) freigegeben wird, wenn zusätzlich das entsprechende Kennwort eingegeben wird, und
bei einer erstmaligen Anmeldung an dem Medizingerätesteuerungssystem ein Lang-Kennwort, das umfangreicher als das Kennwort ist, eingegeben wird, das Medizingerätesteuerungssystem nach einer vorbestimmten Zeitdauer, in dem die mobile Steuerungseinheit (3) nicht bedient wird, die Freigabe aufhebt, und dann eine Anmeldung mit der Eingabe des Kennworts erfolgt.

2. Verfahren gemäß Anspruch 1, wobei, wenn Signale mit jeweiligen Identifikationsmerkmalen von mehreren Funk-Sendemodulen (5) durch das Funk-Empfangsmodul (4) empfangen werden, den Identifikationsmerkmalen von sämtlichen Signalen entsprechende Anzeigen an der mobilen Steuerungseinheit (3) ausgegeben werden und die Bedienfunktion der mobilen Steuerungseinheit (3) freigegeben wird, wenn eine der Anzeigen ausgewählt wird und das entsprechende Kennwort eingegeben wird.

3. Verfahren gemäß einem der Ansprüche 1 oder 2 mit einem Medizingerätesteuerungssystem, bei dem zusätzlich das Funk-Empfangsmodul (4) so angepasst ist, dass es eine Signalstärke des Funksignals erfasst, und dass es einen Schwellwert für die Signalstärke aufweist, wobei nur Funksignale, deren Signalstärke über dem Schwellwert liegen, weitergeleitet werden, wobei der Schwellwert so eingestellt wird, dass nur die entsprechenden Anzeigen von innerhalb eines vorbestimmten Abstands von der mobilen Steuerungseinheit (3) befindlichen Funk-Sendemodulen (5) gezeigt werden.

4. Verfahren gemäß einem der Ansprüche 1 bis 3, wobei nur eine dem Identifikationsmerkmal zugeordnete Bedienfunktion freigegeben wird.

## Claims

1. Method for operating a medical apparatus control system comprising
at least one mobile control unit (3), for at least one medical apparatus (2), with at least one radio receiving module (4) for detecting and forwarding radio signals,
a control device (6) connected to the mobile control unit (3), and
at least one separate mobile radio transmitting module (5) enabled to communicate with the radio receiving module (4),
wherein the at least one radio transmitting module (5) is configured to send an identification feature to the radio receiving module (4), and
the control device (6) is configured to detect a signal, having the identification feature, detected by the radio receiving module (4) and forwarded to the control device (6), to compare the identification feature with the stored identification features, and to release an operating function of the mobile control unit (3) after a positive result of comparison, wherein
the at least one radio transmitting module (5) sends a signal having its identification feature,
the radio receiving module (4) receives the signal and forwards it to the control device (6),
the control device (6) compares the identification feature with stored identification features, and
the control device (6) releases the operating function of the mobile control unit (3) if the result of comparison is positive,
**characterized in that**
a respective password is stored for the identification feature of each radio transmitting module (5), and the mobile control unit (3) outputs a prompt corresponding to the identification feature, and releases the operating function of the mobile control unit (3) if the appropriate password is additionally input, and
upon a first login at the medical apparatus control system, a long-password being more extensive than the password is input, the medical apparatus control system reverses the release after a predefined period in which the mobile control unit (3) is not operated, and a login with the input of the password subsequently occurs.

2. Method according to claim 1, wherein, if signals having the respective identification features of several radio transmitting modules (5) are received by the radio receiving module (4), prompts corresponding to the identification features of all of the signals are output on the mobile control unit (3), and the operating function of the mobile control unit (3) is released if anyone of the prompts is selected and the respective password is input.

3. Method according to anyone of the claims 1 or 2 with a medical apparatus control system in which the radio receiving module (4) is additionally adapted such that it detects a signal strength of the radio signal, and that it comprises a threshold for the signal strength, wherein only radio signals, the signal strength of which is above the threshold, are forwarded, wherein the threshold is adjusted such that only the respective prompts of radio transmitting modules (5) located within a predefined distance from the mobile control unit (3) are shown.

4. Method according to anyone of the claims 1 to 3, wherein only an operating function assigned to the identification feature is released.

## Revendications

1. Procédé pour faire fonctionner un système de commande d'appareils médicaux, comprenant
au moins une unité de commande mobile (3) pour au moins un appareil médical (2) avec au moins un module de réception radio (4) pour détecter et transmettre des signaux radio,
un dispositif de commande (6) relié à l'unité de commande mobile (3), et
au moins un module d'émission radio mobile séparé (5), qui est capable de communiquer avec le module de réception radio (4),
où le au moins un module d'émission radio (5) est conçu pour émettre une caractéristique d'identification pour le module de réception radio (4), et
le dispositif de commande (6) est conçu pour détecter un signal avec la caractéristique d'identification, détecté par le module de réception radio (4) et transmis au dispositif de commande (6), comparer la caractéristique d'identification avec des caractéristiques d'identification mémorisées et valider une fonction d'utilisation de l'unité de commande mobile (3) après un résultat de comparaison positif, où
le au moins un module d'émission radio (5) émet un signal avec sa caractéristique d'identification,
le module de réception radio (4) reçoit le signal et le transmet au dispositif de commande (6),
le dispositif de commande (6) compare la caractéristique d'identification avec des caractéristiques d'identification mémorisées, et
le dispositif de commande (6) valide la fonction d'utilisation de l'unité de commande mobile (3) si le résultat de la comparaison est positif,
**caractérisé en ce qu'**un mot de passe correspondant est mémorisé pour la caractéristique d'identification de chaque module d'émission radio (5), et l'unité de commande mobile (3) affiche une indication correspondant à la caractéristique d'identification et la fonction d'utilisation de l'unité de commande mobile (3) est validée si en outre le mot de passe correspondant est introduit, et
lors d'une première demande au système de commande d'appareils médicaux, un mot de passe long qui est plus étendu que le mot de passe est introduit, le système de commande de dispositifs médicaux supprime la validation après une période de temps prédéterminée au cours de laquelle l'unité de commande mobile (3) n'est pas utilisée, puis a lieu une demande avec l'introduction du mot de passe.

2. Procédé selon la revendication 1, dans lequel, lorsque des signaux avec des caractéristiques d'identification respectives de plusieurs modules de transmission radio (5) sont reçus par le module de réception radio (4), des indications correspondant aux caractéristiques d'identification de tous les signaux sont affichées sur l'unité de commande mobile (3) et la fonction d'utilisation de l'unité de commande mobile (3) est validée si l'une des indications est choisie et le mot de passe correspondant est introduit.

3. Procédé selon l'une des revendications 1 ou 2 avec un système de commande de dispositifs médicaux dans lequel le module de réception radio (4) est en outre adapté de telle sorte qu'il détecte une intensité de signal du signal radio et qu'il présente une valeur seuil pour l'intensité du signal, dans lequel seuls les signaux radio dont l'intensité de signal est supérieure à la valeur seuil sont transmis, dans lequel la valeur seuil est établie de telle sorte que seules les indications correspondantes de modules d'émission radio (5) situés à une distance prédéterminée de l'unité de commande mobile (3) sont représentées.

4. Procédé selon l'une des revendications 1 à 3, dans lequel seule une fonction d'utilisation affectée à la caractéristique d'identification est validée.
